# EUROPÄISCHE PATENTSCHRIFT

(11) **EP 0 536 564 B1**
(45) Veröffentlichungstag und Bekanntmachung des Hinweises auf die Patenterteilung: **16.07.1997**
(21) Anmeldenummer: 92115737.6
(22) Anmeldetag: 15.09.1992
(51) Int. Cl.: C07K 1/00, C07K 14/315, A61K 39/09

(54) **Verfahren zur Reinigung von Streptolysin 0, intaktes Streptolysin 0 erhältlich nach diesem Verfahren und seine Verwendung**
Method to purify streptolysin O, intact streptolysin O obtainable by this method and its use
Méthode de purification de streptolysine O, streptolysine O intacte obtenue par cette méthode et son utilisation

(30) Priorität: 11.10.1991 DE 4133707
(43) Veröffentlichungstag der Anmeldung: 14.04.1993
(73) Patentinhaber: BEHRINGWERKE Aktiengesellschaft, 35001 Marburg (DE)
(72) Erfinder: Althaus, Harald, W-3552 Wetter (DE); Merle, Peter, W-3550 Marburg 9 (DE)

(56) Entgegenhaltungen:
- WO-A-93/05152
- WO-A-93/05156
- GB-A- 2 233 977
- METHODS IN ENZYMOLOGY (MICROBIAL TOXINS: TOOLS IN ENZYMOLOGY) Bd. 165 , 1988 Seiten 52 - 59 ALOUF, J., ET AL. 'Production, purification, and assay of Streptolysin O'
- DATABASE WPI Section Ch, Week 9002, Derwent Publications Ltd., London, GB; Class B04, AN 90-011073 & JP-A-1 290 698 (FUJI REBIO) 22. November 1989
- C. R. ACAD. SC. PARIS SERIE D Bd. 287 , 23. Oktober 1978 Seiten 951 - 954 PRIGENT, D., ET AL. 'Purification de la streptolysine O par chromatographie covalente sur gel de thiol-agarose'
- THE BIOCHEMICAL JOURNAL Bd. 207, Nr. 3 , 1. Dezember 1982 Seiten 557 - 560 JOHNSON, M.K., ET AL. 'The hydrophobic character of thiol-activated cytolysins'
- INFECTION AND IMMUNITY Bd. 55, Nr. 12 , Dezember 1987 Seiten 3228 - 3232 KEHOE, M.A., ET AL. 'Nucleotide sequence of the strptolysin O (SLO) gene: Structural homologies between SLO and other membrane-damaging, thiol-activated toxins'
- METHODS IN ENZYMOLOGY (GUIDE TO PROTEIN PURIFICATION) Bd. 182 , 1990 Seiten 392 - 421 CHICZ, R., ET AL. 'High-performance liquid chromatography: Effective protein purification by various chromatographic modes'
- INFECTION AND IMMUNITY Bd. 46, Nr. 2 , November 1984 Seiten 394 - 400 BHAKDI, S., ET AL. 'Isolation and identification of two hemolytic forms of streptolysin O'

## Beschreibung

Die Erfindung betrifft ein Verfahren zur Reinigung von Streptolysin O (SLO) mittels Chromatographie sowie die Verwendung von Streptolysin.

Streptolysin O (nachfolgend bezeichnet als SLO) ist ein extrazelluläres, cytolytisches Toxin, das von Bakterien der Gattung Streptococcus gebildet wird. Bei der Infektion des Menschen durch Streptococcen werden Antikörper gegen SLO gebildet, denen eine diagnostische Bedeutung zukommt.

Aufgrund proteolytischer Aktivität in den Kulturen von Streptococcen ist es bisher nicht gelungen, größere Mengen des intakten SLO mit dem Molekulargewicht von 60000 D zu isolieren. Es wird überwiegend ein proteolytisch degradiertes SLO vom Molekulargewicht um 53000 D isoliert. Es ist auch bekannt, daß SLO durch Luftsauerstoff reversibel inaktiviert und durch Thiolverbindungen aktiviert werden kann. Alle zur Reinigung von SLO bekannten Verfahren sind sehr aufwendig, enthalten kostspielige Verfahrensschritte und liefern nur geringe Ausbeuten an hochreinem Material, so daß sie für die Reinigung von SLO im Produktionsmaßstab ungeeignet sind. In Biochem. J. 207, 557-560 (1982) ist beschrieben, daß es nicht gelungen ist, SLO mittels hydrophober Interaktionschromatographie in aktiver Form zu gewinnen.

Überraschend wurde gefunden, daß es möglich ist, SLO beispielsweise aus einer Kulturlösung in Gegenwart einer wasserlöslichen Thiolverbindung und einer ausreichend hohen Konzentration von Ammoniumsulfat an Phenyl-^{R}Sepharose oder einen anderen zur hydrophoben Interaktionschromatographie geeigneten Träger in einem Puffer zu binden und durch Gradientenelution in einem absteigenden Ammoniumsulfatgradienten von anderen Proteinkomponenten abzutrennen.

Gegenstand der Erfindung ist daher ein Verfahren zur Reinigung von Streptolysin O (SLO), dadurch gekennzeichnet, daß man eine SLO enthaltende Lösung in Gegenwart einer wasserlöslichen Thiolverbindung und eines Salzes mit einem zur hydrophoben Interaktionschromatographie geeigneten Material in einem Puffer in Kontakt bringt und durch Gradientenelution mit einer Lösung eines Salzes mit negativem Konzentrationsgradienten, der mit einer Konzentration beginnt, die kleiner als die Konzentration des Salzes bei der Adsorption ist, eluiert und die Fraktion, die das SLO enthält, gewinnt.

Mit diesem einfachen Verfahren gelingt es, in hohen Ausbeuten biologisch hochaktives SLO, das weitgehend frei von Begleitenzymen ist, zu isolieren. Im Gegensatz zu den in der Literatur beschriebenen SLO-Produkten ist das nach dem vorliegenden Verfahren gereinigte SLO nicht proteolytisch fragmentiert, also intaktes SLO.

Die SLO enthaltende Lösung kann ein Kulturkonzentrat von Streptococcen, vorzugsweise hämolysierenden Streptococcen sein.

Das SLO in einer solchen Lösung kann aber auch ein gentechnisch hergestelltes Produkt sein. Dieses kann die natürliche oder eine allele Struktur haben oder auch ein Derivat sein.

Gegenstand der Erfindung ist deshalb auch intaktes Streptolysin O erhältlich nach dem beschriebenen Verfahren.

Das bei der Adsorption verwendete Salz ist vorzugsweise Ammoniumsulfat. Es kann jedoch jedes andere Salz, welches auf Proteine einen aussalzenden Effekt zeigt, verwendet werden. Die Konzentration liegt im Falle von Ammoniumsulfat zwischen 15 % und 25 % der Sättigungskonzentration.

Die Thiolverbindung ist eine Protein-Disulfid-Bindungen spaltende Verbindung, vorzugsweise β-Mercaptoethanol, Thioglycerin, Thioglycolat oder Dithiothreitol.

Der zur Chromatographie eingesetzte Puffer ist vorzugsweise ein basischer, vorzugsweise von pH 7,5 - 8,5, vorzugsweise 0,1 M NaHCO₃.

Das Chromatographie-Material enthält vorzugsweise auf der Oberfläche Alkyl- oder Arylgruppen und ist vorzugsweise Phenyl-^{R}Sepharose.

Die nach diesem Verfahren erhaltene Lösung von SLO kann noch durch proteolytisch abgebautes SLO und durch inaktives SLO kontaminiert sein.

Überraschend konnte gefunden werden, daß es möglich ist, aus einer solchen Lösung durch Gelpermeations-Chromatographie in Gegenwart einer Thiolverbindung beispielsweise an Sephacryl^{R} S300 HR oder einem anderen in seiner Trenncharakteristik ähnlichen Chromatographiematerial die inaktiven oder proteolytisch abgebauten SLO-Moleküle zu entfernen und hochaktives intaktes SLO zu gewinnen.

Die Thiolverbindung in der Gelchromatographie ist ebenfalls vorzugsweise eine Protein-Disulfid-Bindungen spaltende Verbindung und besonders β-Mercaptoethanol, Thioglycerin, Thioglycolat oder Dithiothreitol.

Vorzugsweise wird in einem Puffer mit basischem pH-Wert, vorzugsweise von pH 7,5 - 8,5, vorzugsweise in 0,1 M NaHCO₃ gearbeitet.

Zusätzlich kann im Chromatographie-Puffer ein Salz, vorzugsweise Kochsalz, vorzugsweise in einer Konzentration von 0,2 M - 2 M enthalten sein.

Nach diesem Chromatographieschritt liegt SLO als hochreines und hämolytisch hochaktives Präparat vor, das steril und kühl gelagert über Monate stabil bleibt.

Als amino-terminale Aminosäuresequenz wurde NH2-Asp-Ser-Asn-Lys-Gln-Asn-Thr-Ala-Asn-Thr- gefunden.

Das auf diese Weise hergestellte SLO kann in diagnostischen Systemen, die Antikörper gegen SLO in humanem Serum oder Blut-Plasma bestimmen, als Test-Antigen eingesetzt werden. Aufgrund des hohen Reinheitsgrades des verwendeten SLO ist die Spezifität der Testsysteme besser.

Ferner eignet sich das erfindungsgemäß hergestellte SLO, um mittels Immunaffinitätschromatographie hochspezifische menschliche oder tierische Antikörper gegen SLO zu isolieren, die wiederum in diagnostischen Testsystemen eingesetzt als hochspezifische Standards dienen können.

### Beispiele

### Beispiel 1

a. Herstellung des Ausgangsmaterials
   20 Liter eines Kulturüberstandes von Streptococcus pyogenes H46A wurden hergestellt, wie bei Kiefer, D. et al., Infect. Immun. 13(2), 1976, p. 501 - 512 beschrieben. Der durch Zentrifugation geklärte Kulturüberstand wurde mittels einer Ultrafiltrationsanlage, die mit einem Ultrafilter mit 30kD Ausschlußgrenze belegt war, auf ein Volumen von 120 ml eingeengt.
b. Hydrophobe Interaktionschromatographie
   120 ml Ausgangsmaterial aus Beispiel 1a. wurden mit 0,1 Vol % β-Mercaptoethanol und anschließend mit 40 ml gesättigter Ammoniumsulfat-Lösung unter Rühren versetzt; ein eventuell entstehender Niederschlag wurde abzentrifugiert und verworfen. Das Material wurde auf eine Säule (2,5*30 cm) Phenyl-Sepharose^{R}, die mit 0,1 M NaHCO₃, 25 % Ammoniumsulfat, 0,1 Vol % β-Mercaptoethanol äquilibriert war, aufgetragen.
   In einem Gradientenmischer wurden 400 ml des Äquilibrierungspuffers vorgelegt. Es wurde mit einem absteigenden, linearen Ammoniumsulfatgradienten (25 % auf 0 %) in 0,1 M NaHCO₃, 0,1 Vol % β-Mercaptoethanol eluiert.
   Das Eluat wurde fraktioniert aufgefangen.
   Die Fraktionen, die die höchste hämolytische Aktivität zeigten (Bestimmung siehe Beispiel 2) wurden vereinigt und auf ein Maximalvolumen von 10 ml Rohstreptolysin an einer Ultrafilteranlage mit 30kD Ausschlußgrenze eingeengt.

### Beispiel 2

### Gelpermeationschromatographie

Das Rohstreptolysin aus Beispiel 1 wurde auf eine Chromatographiesäule (2,5*80 cm) Sephacryl^{R} S-300 HR aufgetragen, die mit 0,1 M NaHCO₃, 1 M NaCl, 0,1 Vol % β-Mercaptoethanol äquilibriert war.
Es wurde mit Äquilibrierungspuffer unter einer Flußrate von 40 cm/h eluiert, das Eluat wurde fraktioniert aufgefangen.
Die Fraktionen mit der höchsten hämolytischen Aktivität wurden vereinigt und stellen hochreines SLO dar.
Aus 20 l Ausgangsmaterial nach a. konnten 27 mg SLO isoliert werden.

### Analytik:

Eine SDS-Polyacrylamid-Elektrophorese von SLO im Vergleich zu verschiedenen Proteinen mit bekanntem Molekulargewicht (Standard Nr. 4, Serva; Gelsystem nach Lämmli mit 10 % Acrylamid und 0,33 % N,N-Methylen-Bis-acryl-Amid) wurde durchgeführt; die Proteinbanden wurden mit ^{R}Servablau R250 angefärbt.
Das SLO aus Beispiel 2 stellt sich als eine Bande mit dem apparenten Molekulargewicht von ca. 78000 D dar.

### Amino-terminale Proteinsequenzanalyse des SLO aus Beispiel 2

Vom SLO aus Beispiel 2 konnten in einer Sequenzanalyse nach Edman die letzten 10 Aminosäuren des aminoterminalen Proteinendes bestimmt werden. Es wurde die Sequenz NH2-Asp-Ser-Asn-Lys-Gln-Asn-Thr-Ala-Asn-Thr-gefunden.

Die gefundene Sequenz ist in 8 Positionen in Einstimmung mit der aus der DNA-Sequenz von Kehoe, M. et al. (Infect.Immun. 55 (12), 1987, p. 3228-3232) abgeleiteten amino-terminalen Sequenz von SLO. Somit kann gezeigt werden, daß es sich bei dem vorliegenden Produkt um amino-terminal intaktes SLO handelt.

### Bestimmung der hämolytischen Aktivität von SLO

Eine geometrische Verdünnungsreihe des zu prüfenden SLO-haltigen Präparates in PBS, pH 7,2 das 1 % (w/v) BSA und 0,1 Vol % β-Mercaptoethanol enthält wird in einer 96-Loch Microtiterplatte mit U-Profil vorgelegt (100 µl). In jedes Loch der Microtiterplatte werden 100 µl einer 1 %igen (Vol %) frisch hergestellten Kanichen-Erythrozyten-Suspension pipettiert.
Es wird durch leichtes Schütteln gemischt.
Die Platte wird 60 min. bei 37°C in einer feuchten Kammer inkubiert. Danach wird die hämolytische Aktivität durch visuelle Beurteilung bestimmt. Als Titer wird die reziproke Verdünnung der SLO-haltigen Lösung angegeben, die nach der angegebenen Inkubationszeit 50 % der Erythrozyten im Ansatz lysiert.
1 HU (hämolytische Einheit) ist die Menge SLO, die unter den angegebenen Testbedingungen 50 % der eingesetzten Erythrozyten lysiert.

| Fraktion | spezifische Aktivität (HU/mg) |
|---|---|
| Ausgangsmaterial | 12190 |
| SLO hochrein | 512000 |

### Beispiel 3

### Reinigung von Antikörpern gegen SLO mittels Immunaffinitätschromatographie

50 mg eines nach Beispiel 2 gereinigten SLO wurde an mit Cyanbromid aktivierte Sepharose^{R} 4B (20 ml) gekoppelt. Die SLO-Sepharose^{R} wurde in eine Glas-Säule gefüllt und mit PBS, pH 7,2 äquilibriert.
Die Säule wurde mit 30 ml ^{R}Beriglobin (Human-Immunglobulin-Konzentrat 16 %ig) verdünnt mit 90 ml PBS, pH 7,2 beladen. Nicht gebundene Antikörper wurden mit PBS, pH 7,2 von der Säule gewaschen. Der spezifische anti-SLO-Antikörper wurde mit 1 Vol % Essigsäure in Aqua dest. von der Säule eluiert, sofort mit verdünnter Natronlauge neutralisiert und durch Ultrafiltration eingeengt. Aus 30 ml Beriglobin konnten 24,6 mg anti-SLO-Antikörper isoliert werden.

In einer Immundiffusion nach Ouchterlony des gereinigten humanen anti-SLO-Antikörpers gegen SLO-Ausgangsmaterial nach Beispiel 1, Roh-SLO nach Beispiel 1 und gereinigtem SLO nach Beispiel 2 im Vergleich zu ^{R}Beriglobin zeigt ^{R}Beriglobin gegen verschiedene Komponenten des SLO-Ausgangsmaterials Präzipitat-Banden während der gereinigte anti-SLO-Antikörper nur noch mit SLO reagiert.

## Patentansprüche

1. Verfahren zur Reinigung von Streptolysin O (SLO), dadurch gekennzeichnet, daß man eine SLO enthaltende Lösung in Gegenwart einer wasserlöslichen Thiolverbindung und eines Salzes mit einem zur hydrophoben Interaktionschromatographie geeigneten Material in einem Puffer mit einem pH von mehr als 7.5 in Kontakt bringt und durch Gradientenelution mit einer Lösung eines Salzes mit negativem Konzentrationsgradienten, der mit einer Konzentration beginnt, die kleiner als die Konzentration des Salzes bei der Adsorption ist, eluiert und die Fraktion, die das SLO enthält, gewinnt und gegebenenfalls weiter reinigt.

2. Verfahren nach Anspruch 1, dadurch gekennzeichnet, daß die SLO enthaltende Lösung ein Kulturkonzentrat von Streptococcen ist.

3. Verfahren nach Anspruch 1, dadurch gekennzeichnet, daß die SLO enthaltende Lösung ein Kulturkonzentrat von hämolysierenden Streptococcen ist.

4. Verfahren nach Anspruch 1, dadurch gekennzeichnet, daß die SLO enthaltende Lösung ein gentechnisch hergestelltes Produkt ist.

5. Verfahren nach Anspruch 1, dadurch gekennzeichnet, daß ein Salz, welches zum Aussalzen von Proteinen geeignet ist, verwendet wird.

6. Verfahren nach Anspruch 1, dadurch gekennzeichnet, daß die Thiolverbindung eine Protein-Disulfid-Bindungen spaltende Verbindung ist.

7. Verfahren nach Anspruch 1, dadurch gekennzeichnet, daß die Thiolverbindung β-Mercaptoethanol, Thioglycerin, Thioglycolat oder Dithiothreitol ist.

8. Verfahren nach Anspruch 1, dadurch gekennzeichnet, daß der Puffer ein basischer ist.

9. Verfahren nach Anspruch 1, dadurch gekennzeichnet, daß das Chromatographie-Material auf der Oberfläche Alkyl- oder Arylgruppen trägt.

10. Verfahren nach Anspruch 1, dadurch gekennzeichnet, daß die Fraktion, die das SLO enthält durch Gelpermeations-Chromatographie in Gegenwart einer Thiolverbindung weiter gereinigt wird.

11. Verfahren nach Anspruch 10, dadurch gekennzeichnet, daß in einem Puffer, der ein Salz in einer Konzentration von 0,2 M - 2 M enthält, gearbeitet wird.

## Claims

1. A process for the purification of streptolysin O (SLO), wherein a SLO-containing solution is brought into contact, in the presence of a water-soluble thiol compound and a salt, with a material suitable for hydrophobic interaction chromatography, in a buffer having a pH of more than 7.5, and eluted by gradient elution with a solution of a salt using a negative concentration gradient, which starts at a concentration which is lower than the concentration of the salt for the adsorption, and the fraction which contains the SLO is obtained and optionally further purified.

2. The process as claimed in claim 1, wherein the SLO-containing solution is a culture concentrate of streptococci.

3. The process as claimed in claim 1, wherein the SLO-containing solution is a culture concentrate of hemolytic streptococci.

4. The process as claimed in claim 1, wherein the SLO-containing solution is a product prepared by genetic engineering.

5. The process as claimed in claim 1, wherein a salt which is suitable for salting out proteins is used.

6. The process as claimed in claim 1, wherein the thiol compound is a compound which cleaves protein disulfide bonds.

7. The process as claimed in claim 1, wherein the thiol compound is β-mercaptoethanol, thioglycerol, thioglycolate or dithiothreitol.

8. The process as claimed in claim 1, wherein the buffer is an alkaline buffer.

9. The process as claimed in claim 1, wherein the chromatography material carries alkyl or aryl groups on the surface.

10. The process as claimed in claim 1, wherein the fraction which contains the SLO is further purified `by gel permeation chromatography in the presence of a thiol compound.

11. The process as claimed in claim 10, wherein the procedure is carried out in a buffer which contains a salt in a concentration of 0.2 M - 2 M.

## Revendications

1. Procédé de purification de la Streptolysine O (SLO), caractérisé en ce qu'on met en contact, dans un tampon à un pH supérieur à 7,5, une solution contenant de la SLO, en présence d'un dérivé de thiol soluble dans l'eau et d'un sel, avec un matériau convenant pour la chromatographie d'interaction hydrophobe, on élue par élution avec gradient avec une solution d'un sel à gradients de concentration négatifs, commençant avec une concentration, qui est inférieure à la concentration du sel lors de l'adsorption, et on recueille la fraction, qui contient la SLO, et éventuellement on la purifie ultérieurement.

2. Procédé selon la revendication 1, caractérisé en ce que la solution contenant la SLO est un concentré de culture de streptocoques.

3. Procédé selon la revendication 1, caractérisé en ce que la solution contenant la SLO est un concentré de culture de streptocoques hémolytiques.

4. Procédé selon la revendication 1, caractérisé en ce que la solution contenant la SLO est un produit préparé par génie génétique.

5. Procédé selon la revendication 1, caractérisé en ce qu'on utilise un sel, qui convient pour le relargage des protéines.

6. Procédé selon la revendication 1, caractérisé en ce que le dérivé de thiol est un dérivé clivant les liaisons disulfure des protéines.

7. Procédé selon la revendication 1, caractérisé en ce que le dérivé de thiol est le β-mercaptoéthanol, le thioglycérol, le thioglycolate ou le dithiothréitol.

8. Procédé selon la revendication 1, caractérisé en ce que le tampon est un tampon basique.

9. Procédé selon la revendication 1, caractérisé en ce que le matériau pour chromatographie porte à sa surface des groupes alkyle ou aryle.

10. Procédé selon la revendication 1, caractérisé en ce qu'ensuite on purifie la fraction, qui contient la SLO, par chromatographie par perméation de gel en présence d'un dérivé de thiol.

11. Procédé selon la revendication 10, caractérisé en ce qu'on travaille dans un tampon, qui contient un sel à une concentration de 0,2 à 2 M.
